# EUROPEAN PATENT APPLICATION

(11) **EP 1 814 051 A2**
(43) Date of publication of application: **01.08.2007**
(21) Application number: 07250332.9
(22) Date of filing: 26.01.2007
(51) Int. Cl.: G06F 19/00, A63B 69/00, A63B 69/16, G01C 22/00, A63B 22/00

(54) **Remote biometric/positional feedback system**

(30) Priority: 26.01.2006 US 762453 P; 24.01.2007 US 626740
(71) Applicant: Saris Cycling Group, Inc., Madison WI 53711 (US)
(72) Inventor: Watson, Edward M., Madison, Wisconsin 53711 (US)
(74) Representative: Hedges, Martin Nicholas

(57) **Abstract**

A system for gathering and communicating information relative to a person includes sensors associated with the person for sensing characteristics relative to movement of a person over a time period, and a communication arrangement for communicating data representative of the one or more characteristics during the time period to a remote location. The communication arrangement also communicates feedback information to the person from the remote location, based on the sensed characteristics. The feedback may be based on an analysis of the sensed characteristics performed at the remote location. The sensors are interconnected with the person or with an item of equipment carried or operated by the person, and may be operable to sense characteristics such as heart rate, breathing rate, applied power, speed, pace, distance traveled, energy expenditure, and position.

## Description

This application claims the benefit of United States provisional patent application serial number 60/762,453 filed January 26, 2006.

### BACKGROUND AND SUMMARY OF THE INVENTION

This invention relates to a system for monitoring and communicating information about certain characteristics relative to a person.

It is known to detect or monitor certain characteristics relative to movement of a person over time. In a typical application, information pertaining to the detected or monitor characteristics is stored, and is then used at a later time for analysis and/or planning purposes. For example, when a bicycle is being ridden, such as during a race or training session, it is common to monitor and record information such as speed, cadence, heart rate, power output, energy expenditure and the like. The bicycle typically includes a display on which information pertaining to the sensed characteristics is communicated to the rider in a near real-time environment. After conclusion of the race or training session, the information pertaining to the sensed characteristics is then used for analyzing the race or training session, and for planning future race strategies or training sessions. This use of information pertaining to the sensed characteristics has been found to provide benefits in training as well as in the manner in which a rider strategically approaches a race. However, there is a significant time lag between the time the information pertaining to the sensed characteristics is gathered and the time during which the rider can employ the training or race strategies that are developed as a result of analysis of the information.

It is an object of the present invention to provide a system for monitoring and communicating information relative to movement of a person over time, which is capable of immediately communicating such information to a remote location. It is another object of the invention to provide such a system which enables use of the information at the remote location, and communication of feedback information back to the person based on the use of the information at the remote location. Yet another object of the invention is to provide such a system that is capable of operating in a real-time or near real-time manner, so that the person can have feedback information that can be used immediately in order to adjust or alter certain behavior in an ongoing manner, e.g. during a race or training session. A still further object of the invention is to provide such a system which takes advantage of known monitoring and sensing technology, and enables immediate analysis and feedback based on use of information pertaining to the sensed characteristics.

In accordance with the present invention, a system for gathering and communicating information relative to a person includes one or more sensors associated with the person for sensing characteristics relative to movement of a person over a time period, and a communication arrangement for communicating data representative of the one or more characteristics during the time period to a location remote from the person. The communication arrangement is also preferably configured and arranged to communicate information back to the person from the remote location, and the information communicated back to the person from the remote location is preferably in the form of feedback based on the sensed characteristics. In a preferred form, the feedback is based upon an analysis of the sensed characteristics performed at the remote location. Representatively, the data is communicated to and from the remote location by a communication system selected from the group consisting of cellular telephony, wireless internet transmission, and satellite communication. The sensors are preferably associated with the person by interconnecting the sensors with the person or with an item of equipment carried or operated by the person. The sensors are operable to sense one or more of the following characteristics relative to movement of the person: heart rate, breathing rate, applied power, speed, pace, distance traveled, energy expenditure, and position.

The invention also contemplates a method of gathering and communicating information relative to a person to and from a remote location, substantially in accordance with the foregoing summary.

Various other features, objects and advantages of the invention will be made apparent from the following description taken together with the drawings.

### BRIEF DESCRIPTION OF THE DRAWINGS

The drawings illustrate the best mode presently contemplated of carrying out the invention.

In the drawings:
FIG. 1 is a schematic diagram illustrating the information gathering and communication system in accordance with the present invention;
FIG. 2 is a block diagram illustrating components incorporated in the local CPU;
FIG. 3 is a block diagram illustrating the flow of data to and from the local CPU; and
FIG. 4 is a schematic pictorial view of a representative application for the information gathering and communication system in accordance with the present invention, in the form of a bicycle.

### DETAILED DESCRIPTION OF THE INVENTION

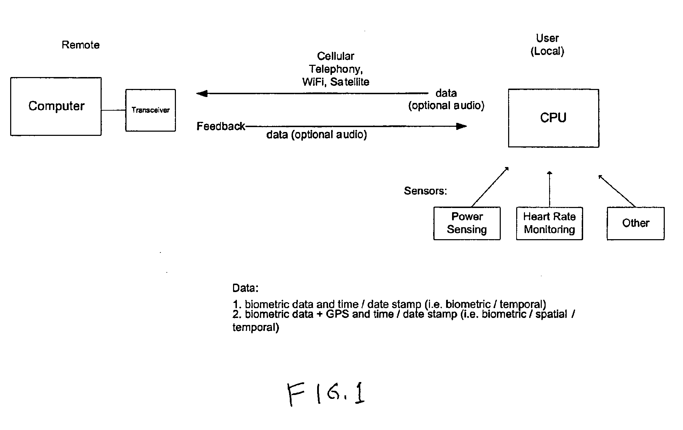

In accordance with the invention, and as shown in FIG. 1, certain information relative to a person's biometric characteristics, performance and/or position is gathered and communicated to a remote location. A person at the remote location can thus continuously know various characteristics relevant to the condition, status or performance history of the person whose characteristics, performance and/or position are being monitored. The information can then be used at the remote location to develop feedback information, which is communicated from the remote location back to the person. For example, the information pertaining to the person's biometric characteristics, performance and/or position may be analyzed using certain criteria and/or parameters, in order to formulate recommended alterations or modifications in the person's movements, power output, performance, etc. that are communicated back to the person in order for the person to achieve a certain result.

The information relative to a person's biometric characteristics, performance and/or position relate to movement of the person over time. Such information may be gathered from sensors carried on, by or interconnected with the person, or on an item of equipment that is with the person, e.g. a bicycle, vehicle, pack, etc. Representatively, the information may include the person's heart rate, breathing rate, power output (relating to movement of the person, either by walking or running, or by operation of a bicycle or other user-propelled device), speed or pace, distance moved, energy expended and position (longitude, latitude and altitude). Heart rate may be gathered by means of a heart rate monitor. Power information may be produced by a power measuring system, such as a hub-type bicycle-mounted power sensor such as is available from Saris Cycling Group, Inc. of Madison, Wisconsin under its designation POWERTAP. Alternatively, power information may be gathered in any other manner. Speed or pace information may be gathered from a speedometer or from a calculation of distance traveled over time. Distance and location information may be gathered using a GPS device worn or carried by the person, or mounted to an item of equipment with the person or that is operated by the person. Distance information may also be gathered using an odometer or other distance measuring device. Work or energy expenditure (typically in Kilojoules) may be determined using an ergometer or other satisfactory device, or may be calculated using power output and time. The person's longitude, latitude and altitude information may be gathered by the GPS unit worn or carried by the person or otherwise associated with the person, as noted above. It is understood that the listed characteristics or parameters are representative of any number of different personal characteristics or parameters that may be sensed or monitored to produce data relevant to a person's condition, status or performance history.

The data collected from the person is recorded in a real-time manner, and also may be analyzed in a real-time manner. The data is then bundled into packets of summary information or averaged per transmission interval. The data packets are then transmitted to a remote receiving station, such as a personal computer. The personal computer may be in a stationary location, or alternatively may be a movable laptop or notebook computer. In any event, the data packets are transmitted from the data gathering location to a location that is remote therefrom, and at which the personal computer receives the data packets. It is understood that the remote location need not be remote in terms of absolute distance, but rather the term "remote" is used to designate a location other than the location at which the information is gathered and from which the information is transmitted.

The data packets may be transmitted, typically via an appropriate wireless infrastructure available for remote reception (e.g. via cellular telephone or text messaging transmission, WiFi transmission, satellite communication, etc). The periodicity of the transmissions is established to provide a contemporaneous picture of the various personal parameters or characteristics that are being sensed or monitored so that, from the remote location, the various characteristics of the person can be ascertained in a close to real-time manner. This information can then be analyzed using the personal computer at the remote location, which may involve an analysis according to certain criteria or parameters. For example, the information relative to the person's biometric and positional characteristics may be analyzed using certain goals, maximum values or minimum values, or ranges. At the remote location, and the information can be provided to a CPU that is capable of performing more complex operations or calculations than can be accomplished at the local CPU, i.e. the CPU that is used on or in connection with the person to gather the sensed information. In addition, the CPU at the remote location may have an Internet connection or access to one or more databases relevant to the sensed characteristics, which means that more data is available at the remote CPU to use and analyze the information than can be stored or handled by the local CPU. From the remote location, feedback information from the analysis can be communicated back to the person. The feedback information may be communicated from a person conducting the analysis at the remote location, or alternatively may be in the form of computerized feedback. The feedback information may be supplied to the person in an audible form, such as via a headset, or may be communicated to a receiver and then displayed on a visual display interconnected with the receiver. The person or computer at the remote location can conduct a rapid analysis of the information, and prompt, near real-time feedback can then be provided to the person as a result of the analysis. The person can then modify or alter his or her movements in order to achieve a desired result.

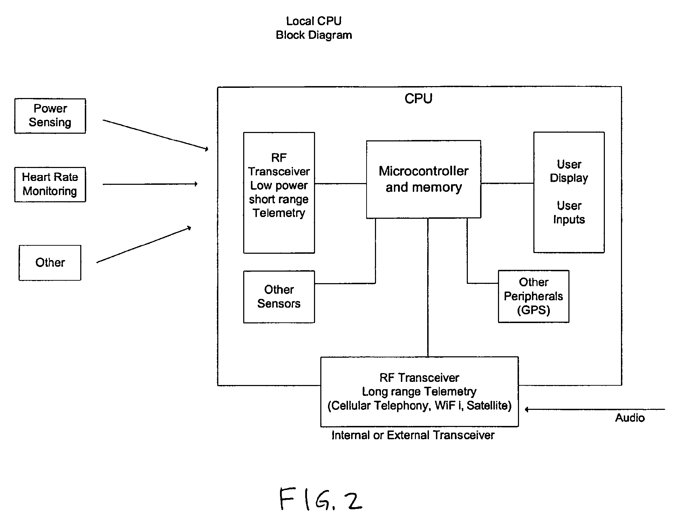

FIG 2 illustrates the components incorporated in the local CPU. The monitored parameters, such as power sensing signals, heart rate signals, etc., are communicated to an RF transceiver, such as via short range telemetry, incorporated in the local CPU. Other sensors, such as speed sensors, ambient condition sensors, etc., may also be incorporated in the local CPU. Information from the transceiver and from the other sensors is supplied to a microcontroller and memory device. Other peripheral devices, such as GPS sensors, are interconnected with and supply information to the microcontroller and memory device. A user interactive display is also interconnected with the microcontroller and memory device, for receiving and displaying information from microcontroller and memory device, and for enabling user inputs to be provided to the microcontroller and memory device. In addition, a long-range RF transceiver is interconnected with the microcontroller and memory device, for transmitting the data to the remote location.

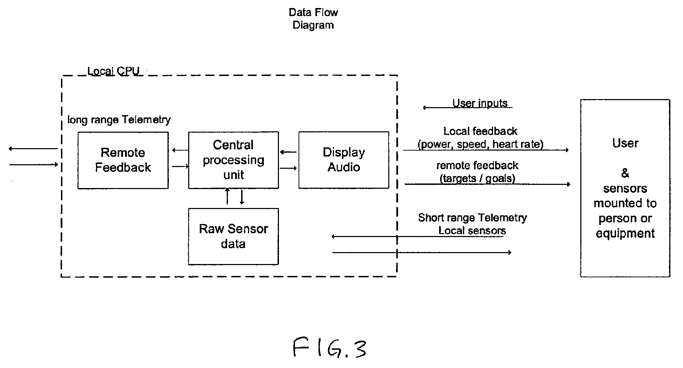

FIG 3 shows the flow of data using the system of the present invention. User input is provided to the local CPU, and information from the sensors mounted to the person or equipment is also provided to the local CPU via short range telemetry. Such information is processed by the local CPU, and is communicated via long range telemetry to the remote location. From the remote location, the remote feedback is supplied to the local CPU. An audio component may be incorporated in the local CPU, for providing audible feedback and also to receive audible inputs from the user. From the local CPU, local feedback information is provided to the user, such as power, speed, heart rate, etc. Remote feedback is also provided to the user from the local CPU, such as targets, goals, etc.

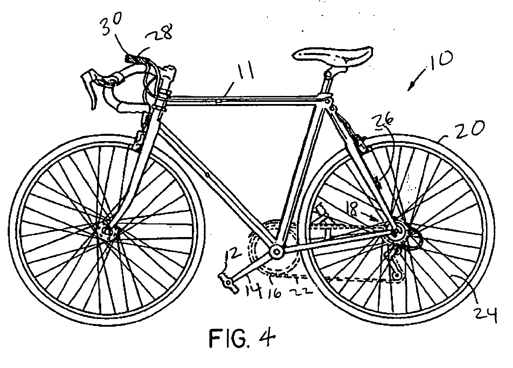

FIG. 4 illustrates a representative application of the information gathering and communication system of the present invention. As shown in FIG. 2, a bicycle 10 includes a frame 11, which rotatably supports a pair of pedals 12 connected by crank arms 14 to a chain ring 16. The chain ring 16 is coupled to the hub assembly 18 of the rear wheel 20 by a chain 22. The bicycle 10 is powered by a rider providing rotational forces to the chain ring 16 via the pedals 12 and crank arms 14. The rotation of the chain ring 16 is transferred by the chain 22 to the rear wheel hub assembly 18, which carries the rear wheel 20 into rotation via spokes 24 to drive the bicycle into motion.

The rear wheel hub assembly 18 has a construction as set forth in Ambrosina et al US patent 6,418,797, and is operable to sense the applied torque in and angular velocity of the hub assembly 18. The detected torque-related and angular velocity related values are sensed by electronic components within the hub assembly 18, and are then transmitted by, for example, radio frequency waves, to a receiver module 26, which is mounted in any satisfactory location on the frame 11 of bicycle 10. The receiver module 26 transmits such information, such as via a wire connection, to a cycle computer 28 which can be mounted on the handlebars of the bicycle 10 or elsewhere on the frame 11. Alternatively, the hub assembly 18 may incorporate a signal transmission system that transmits signals wirelessly to a receiver associated with cycle computer 28. The computer 28 uses the torque-related and angular velocity related information to compute applied power and velocity, and can display the measured or calculated information on a display as desired. In addition, the rider is provided with a heart rate monitor, which representatively may be in the form of a conventional chest strap. The cycle computer 28 includes a receiver that receives the rider's heart rate information transmitted from the chest strap. The cycle computer 28 may also include a GPS transceiver, which functions in a known manner to receive GPS signals for position and location information.

In a bicycle race or training session using bicycle 10, the rider's power, heart rate, speed, distance and position information are supplied by the cycle computer 28 to a communication device, which may be in the form of a cellular telephone, a wireless Internet transmitter, a satellite phone, etc. The communication device may be incorporated in cycle computer 28, or alternatively may be a communication device that is separate from and interconnected with cycle computer 28. As noted above, the cycle computer 28 organizes the information pertaining to the sensed or monitored characteristics into data packets, which are supplied to the communication device. The data packets are then transmitted bv the communication device away from the bicycle 10 to a remote location. In this case, the remote location may be as close as a trailing vehicle of the type that is commonly employed in a bicycle race to trail a rider or team. The vehicle may contain a laptop or notebook computer equipped with a receiver that receives the transmitted information. In the vehicle, the rider's trainer or coach can use the computer to analyze the information as desired, and can then create feedback that can be immediately supply to the rider to alter various performance characteristics as necessary to achieve a desired result. Alternatively, the remote location may be far from the location at which the information is gathered, and in fact may be any location that can be reached using telephonic or wireless Internet communication signals.

Representatively, the data packets communicated to the remote location in the case of bicycle 10 may be comprised of the information as set forth in the following table.

| **info** | **Data (example)** | **Description** |
|---|---|---|
| 1 | 1999 | 0 to 1999 average watts (Power) |
| 2 | 255 | 0 to 255 average bpm (Heart Rate) |
| 3 | 60.0 | 2 to 60.0 average MPH (Speed) |
| 4 | 140 | 40 to 140 average Cadence (RPM) |
| 5 | 00000.00 | Distance traveled (miles) |
| 6 | 00000 | Kilojoules (energy) |
| 7 | 00:00:00 | Time stamp transmission hour:min:sec (active time) |
| 8 | 0007.038N | 00 deg 00.00 minutes N (or S) Latitude |
| 9 | 0007.0 38E | 00 deg 00.00 minutes E (or W) Latitude |
| 1 0 | 00.00.00 | Number satellites. fix info |
| 11 | 000.0 | Altitude above mean sea level (meters) |
| 12 | 00.0 | Height above geoid (mean sea level) |
| 13 | 12:59:59 | UTC GPS location time stamp |

This information can then be analyzed by the coach or manager, who can then communicate the information as well as advice or recommendations back to the rider, e.g. via a mobile communication system, which may involve alterations or modification to performance that may be necessary or desirable to achieve a desired result. Representatively, the data packets communicated to the rider from the remote location in the case of bicycle 10 may be comprised of the information as set forth in the following table.

| **info** | **Data (example)** | **Description** |
|---|---|---|
| 1 | 1999 | 0 to 1999 target watts (Power) |
| 2 | 0:00:00 | Pace time +/-:min:sec (+/- from goal or active race) |
| 3 | 60.0 | 2 to 60.0 target MPH (Speed) |
| 4 | 00000.00 | Distance remaining (miles) |
| 5 | 0:00:00 | Time remaining hour:min:sec (active time) |
| 6 | 0000 | Food units intake (energy input, based on energy expenditures) |
| 7 | 00 | Hydration (based on ambient conditions and biometrics) |

There are numerous other applications for the information gathering and communication system in accordance with the present invention. The following are simply some examples of additional applications in which the information gathering and communication system may be employed, with the understanding that such examples are representative of many other applications. In a distance running application, information relative to a runner's speed, distance traveled, location, breathing rate and heart rate may be communicated to the remote location. Such information can be analyzed at the remote location using various criteria or parameters, such as the runner's target time, running or race route, weather conditions, etc., and feedback information can be provided to the runner as to alterations or modifications that may be necessary to achieve a desired result. In a military application, information relating to a soldier on the ground can be communicated back to a command area, where a person can ascertain the location of the soldier as well as the soldier's current biometric information and history to determine whether the soldier is (or may become) under stress. From the remote location, a person can also monitor the soldier's energy expenditures over time to determine the state of fatigue or other condition of the soldier. The person at the remote location can then provide feedback to the soldier as to modifications or alterations in the soldier's movement, and in order to achieve a desired result. In either a military or non-military application, e.g. in a park service application or the like, a person at the remote location can know the same information as above in the event a park ranger or other person is involved in a search and rescue operation. In an application such as this, positional information is especially important so that personnel can be located in the event it becomes necessary to extract the person, such as due to oncoming fire, weather conditions or the like. There are numerous other applications, not set forth above, in which this technology can be advantageously employed.

Various alternatives and embodiments are contemplated as being within the scope of the following claims particularly pointing out and distinctly claiming the subject matter regarded as the invention.

## Claims

1. A system for gathering and communicating information relative to a person, comprising:
one or more sensors associated with the person for sensing characteristics relative to movement of a person over a time period; and
a communication arrangement for communicating data representative of the one or more characteristics during the time period to a location remote from the person.

2. The system of claim 1, wherein the communication arrangement is configured and arranged to communicate information back to the person from the remote location.

3. The system of claim 2, wherein the information communicated back to the person from the remote location comprises feedback based on the sensed characteristics.

4. The system of claim 3, wherein the feedback is based upon an analysis of the sensed characteristics performed at the remote location.

5. The system of claim 2, wherein the data is communicated to and from the remote location by a communication system selected from the group consisting of cellular telephony, wireless internet transmission, and satellite communication.

6. The system of claim 2, wherein the one or more sensors are associated with the person by interconnecting the one or more sensors with the person or with an item of equipment carried or operated by the person.

7. The system of claim 6, wherein the one or more sensors are operable to sense one or more of the following characteristics relative to movement of the person: heart rate, breathing rate, applied power, speed, pace, distance traveled, energy expenditure, and position.

8. A system for gathering and communicating information relative to a person, comprising:
sensing means associated with the person for sensing characteristics relative to movement of a person over a time period; and
communication means for communicating data representative of the one or more characteristics during the time period to a location remote from the person.

9. The system of claim 8, wherein the communication means is configured and arranged to communicate information back to the person from the remote location.

10. The system of claim 9, wherein the information communicated back to the person from the remote location comprises feedback based on the sensed characteristics.

11. The system of claim 10, wherein the feedback is based upon an analysis of the sensed characteristics performed at the remote location.

12. The system of claim 9, wherein the communication means is selected from the group consisting of cellular telephony, wireless internet transmission, and satellite communication.

13. The system of claim 9, wherein the sensing means is associated with the person by interconnecting the sensing means with the person or with an item of equipment carried or operated by the person.

14. The system of claim 13, wherein the sensing means is operable to sense one or more of the following characteristics relative to movement of the person: heart rate, breathing rate, applied power, speed, pace, distance traveled, energy expenditure, and position.

15. A method of gathering and communicating information, comprising the acts of:
sensing one or more characteristics relative to movement of a person; and
communicating data representative of the one or more characteristics to a location remote from the person.

16. The method of claim 15, including the act of communicating information back to the person from the remote location.

17. The method of claim 16, wherein the act of communicating information back to the person from the remote location comprises communicating feedback information to the person based on the data.

18. The method of claim 17, including the act of analyzing the data at the remote location, and wherein the act of communicating feedback information to the person from the remote location comprises communicating feedback information based on the analysis of the data at the remote location.

19. The method of claim 16, wherein the acts of communicating data representative of the one or more characteristics to a location remote from the person, and communicating information back to the person from the remote location, are carried out by one of cellular telephony, wireless internet transmission, and satellite.

20. The method of claim 16, wherein the act of monitoring the one or more characteristics of the person is carried out by measuring the one or more characteristics directly from the person or by measuring the one or more characteristics from an item of equipment carried by or operated by the person.

21. The method of claim 16, wherein the act of sensing the one or more characteristics relative to movement of the person is carried out by monitoring one or more of the person's heart rate, breathing rate, applied power, speed, pace, distance traveled, energy expenditure, and position.
